(19)

**Europäisches Patentamt**
**European Patent Office**
**Office européen des brevets**

(11) **EP 4 786 976 A1**

(12) **EUROPEAN PATENT APPLICATION**
published in accordance with Art. 153(4) EPC

(43) Date of publication:
**05.08.2026 Bulletin 2026/32**

(21) Application number: **24882027.6**

(22) Date of filing: **04.09.2024**

(51) International Patent Classification (IPC):
**G01N 33/48** (2006.01)     **G01N 1/30** (2006.01)

(52) Cooperative Patent Classification (CPC):
**G01N 1/30; G01N 33/48**

(86) International application number:
**PCT/JP2024/031780**

(87) International publication number:
**WO 2025/088910 (01.05.2025 Gazette 2025/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.10.2023   JP 2023184062**

(71) Applicants:
• **Hitachi High-Tech Corporation**
  **Minato-ku**
  **Tokyo 105-6409 (JP)**

• **Ventana Medical Systems, Inc.**
  **Tucson, Arizona 85755 (US)**

(72) Inventors:
• **SAITO, Hisao**
  **Tokyo 100-8280 (JP)**
• **MATSUBARA, Shigeki**
  **Tokyo 105-6409 (JP)**
• **SUGIYAMA, Hidetoshi**
  **Tokyo 105-6409 (JP)**
• **OBARA, Takayuki**
  **Tokyo 105-6409 (JP)**

(74) Representative: **Strehl & Partner mbB**
**Maximilianstrasse 54**
**80538 München (DE)**

(54) **INFORMATION PROCESSING DEVICE AND INFORMATION PROCESSING METHOD**

(57)     Provided is a technology for presenting at least one heating condition for achieving an optimal staining intensity of a staining target. This information processing device, which is for presenting at least one heating condition for a staining target, includes a memory for storing a program and a processor for reading and executing the program from the memory. The processor performs: processing for reading information relating to a correlation between a staining intensity of a stained specimen and a time integral value of a heating temperature from a storage device that stores the information relating to the correlation, where the information relating to the correlation includes a correlation approximation expression between a staining intensity calculated from a staining image of the stained specimen and a time integral value of a heating temperature, calculated from a heating history of the stained specimen; processing for accepting a selection of a desired staining intensity for the staining target; processing for calculating a time integral value corresponding to the desired staining intensity that has been selected, on the basis of the information relating to the correlation, read from the storage device, and calculating the at least one heating condition of the staining target from the time integral value; and processing for displaying the calculated at least one heating condition on a display device.

FIG. 3

EP 4 786 976 A1

**Description**

[Technical Field]

This disclosure relates to an information processing device and an information processing method.

[Background Art]

To observe a biological tissue or cell through a microscope, a specimen is prepared by fixing the biological tissue with a fixative such as formalin, dehydrating it with ethanol or the like, embedding it in paraffin, and sectioning the tissue with a microtome at approximately 3 to 5 μm. The prepared specimen is placed on a glass slide, deparaffinized, pretreated, and then, stained while utilizing hybridization with a labeled probe or an antigen-antibody reaction with a labeled antibody. The pretreatment is a process that exposes the intracellular target substance and promotes its reaction to an externally supplied reagent (e.g., a labeled antibody); typically, it is heat treatment.

Regarding the heat treatment, the paragraph 0005 of Patent Literature 1 states: For example, an overnight incubation at 60°C or a 1-hour incubation at 95°C have both been reported to be used successfully for pre-IHC AR.

In recent years, in order to reduce the heating time, a further technique has been proposed that uses a pressure heater (autoclave or pressure vessel) that generates high-pressure steam to heat a specimen at 100°C or higher (refer to Non-Patent Literature 1). The paragraph 0008 of Patent Literature 1 states: Christensen et al. disclosed a horizontal AR apparatus and methods where an AR temperature of 120°C to 130°C was obtained with the use of modified buffers.

[Citation List]

[Patent Literature]

[PTL 1]
JP 2020-527707 A
[NPL 1]
Aichi Prefecture Clinical Testing Quality Control Survey, 2014, pp. 217-235

[Summary of Invention]

[Technical Problem]

Traditionally, the typical heat treatment has been incubation at 95°C to 100°C for one hour. Accordingly, there are few examples of staining with a heat treatment at high temperature of 120°C to 130°C using a pressurizing heater; there is a little knowledge regarding appropriate heating conditions (a combination of a heating temperature and a heating time). Heating conditions significantly affect the intensity of the stain of a specimen that has been stained (hereinafter referred to as stained specimen). Insufficient heating may result in poor staining and a false negative. On the other hand, excessive heating may cause tissue breakdown or non-specific staining, resulting in a false positive. Hence, while determination of heating conditions is extremely important, the lack of prior knowledge requires the operator to search for optimal conditions through trial and error. However, the specimen to be stained is more likely to be obtained from a patient's lesion and cannot be replaced, trial and error is not preferable for determining the conditions.

This disclosure has been accomplished in view of the above-described problems and it provides a technique to present heating conditions that attain an optimal staining intensity for a staining target.

[Solution to Problem]

To solve the above problem, an information processing device of this disclosure is an information processing device for presenting at least one heating condition for a staining target, the information processing device including: a memory configured to store a program; and a processor configured to retrieve the program from the memory and execute the program, wherein the processor is configured to perform the processing of: retrieving information about a correlation between a staining intensity of a stained specimen and a time integral value of heating temperature for the stained specimen from a storage device holding the information about the correlation, the information about the correlation including an approximate formula for the correlation between the staining intensity calculated from a stained image of the stained specimen and the time integral value of heating temperature calculated from a heating history of the stained specimen; receiving a selection of a desired staining intensity for the staining target; calculating a time integral value of heating temperature corresponding to the selected desired staining intensity based on the information about the correlation retrieved from the storage device and calculating the at least one heating condition for the staining target from the time integral value; and displaying the calculated at least one heating condition on a display device.

Further features related to this disclosure will be clarified from the description of this specification and the accompanying drawings. The aspects of this disclosure are achieved and implemented by the elements, various combinations of elements, and the aspects of the following detailed description and the appended claims. The description of this specification merely provides typical examples and does not limit the scope of the claims of this disclosure or their applications in any way.

[Advantageous Effects of Invention]

The technique of this disclosure enables presentation of heating conditions that attain an optimal staining intensity for a staining target. Problems, configurations, and effects other than those described above will be clarified by the description of the following embodiments.

[Brief Description of Drawings]

[FIG. 1]

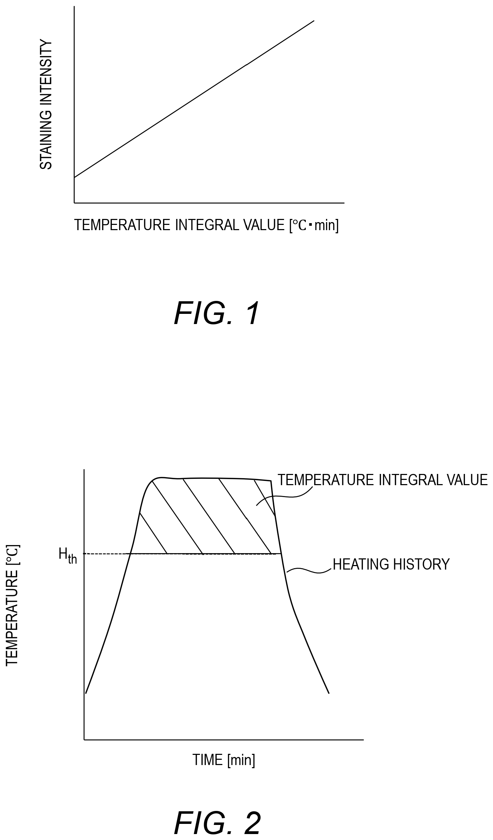

FIG. 1 is a diagram showing the correlation between the intensity of the stain in a stained specimen and the time integral value of the heating history (temperature integral value) in the pre-staining treatment.
[FIG. 2]
FIG. 2 is a diagram for explaining the definition of the temperature integral value.
[FIG. 3]

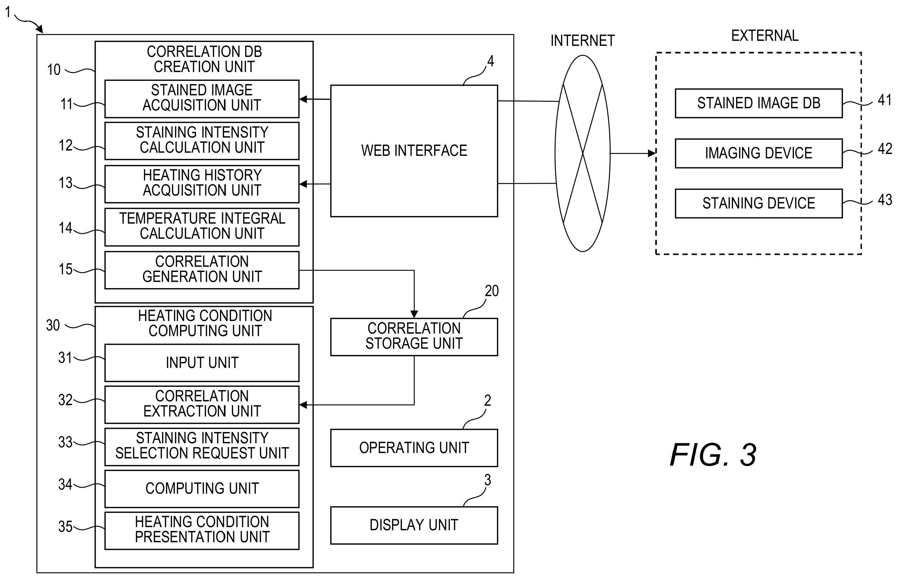

FIG. 3 is a functional block diagram of a heating condition presentation device in the first embodiment.
[FIG. 4]

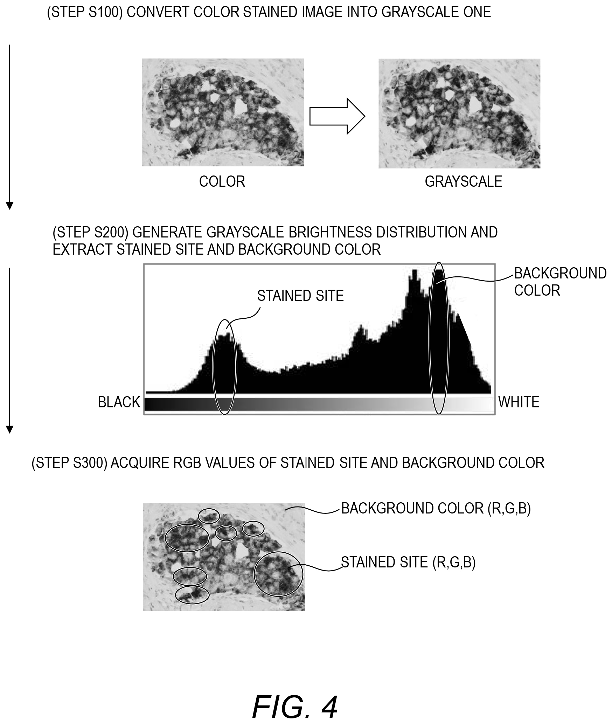

FIG. 4 is a diagram for explaining image processing of a staining intensity calculation unit.
[FIG. 5]

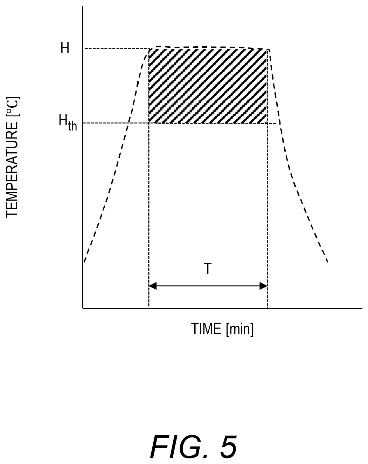

FIG. 5 is a diagram for explaining the temperature integral value in the case of acquiring heating conditions from a stained image database.
[FIG. 6A]

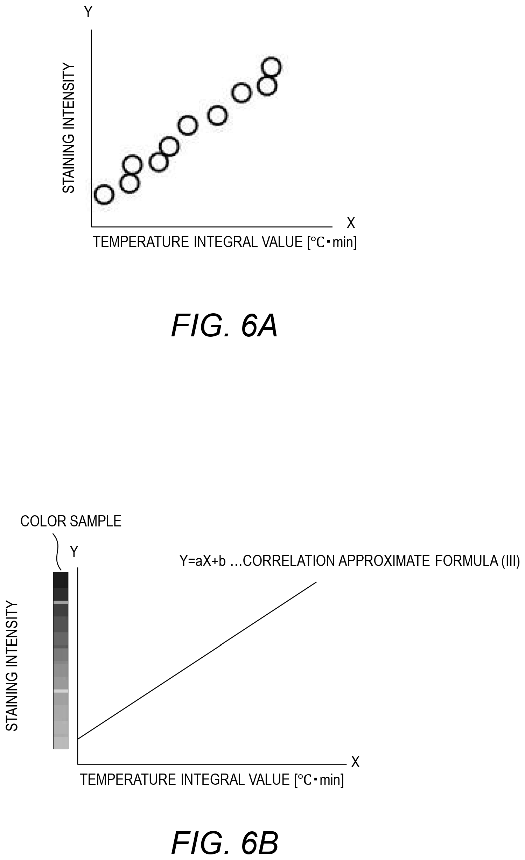

FIG. 6A is a diagram for explaining processing of a correlation generation unit.
[FIG. 6B]
FIG. 6B is a diagram for explaining processing of the correlation generation unit.
[FIG. 7]

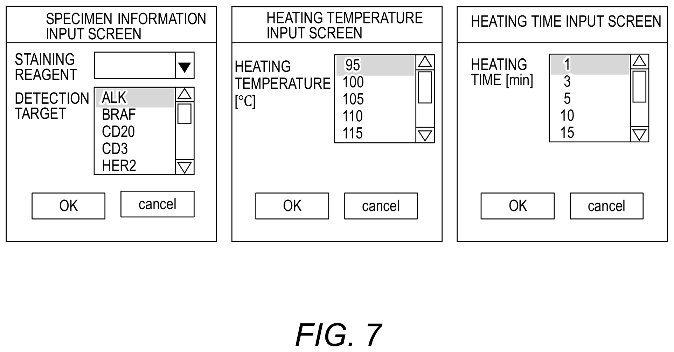

FIG. 7 is a diagram for explaining processing of an input unit.
[FIG. 8]

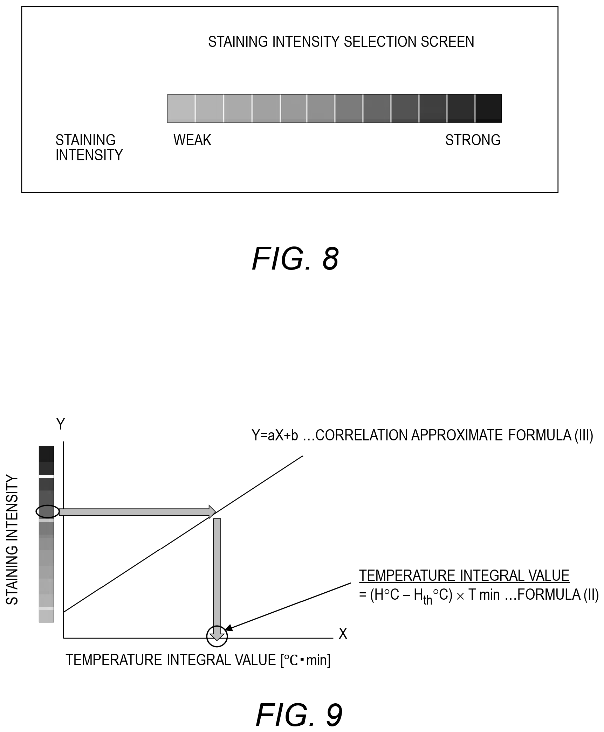

FIG. 8 is a diagram for explaining processing of a staining intensity selection request unit.
[FIG. 9]
FIG. 9 is a diagram for explaining processing of a computing unit.
[FIG. 10]

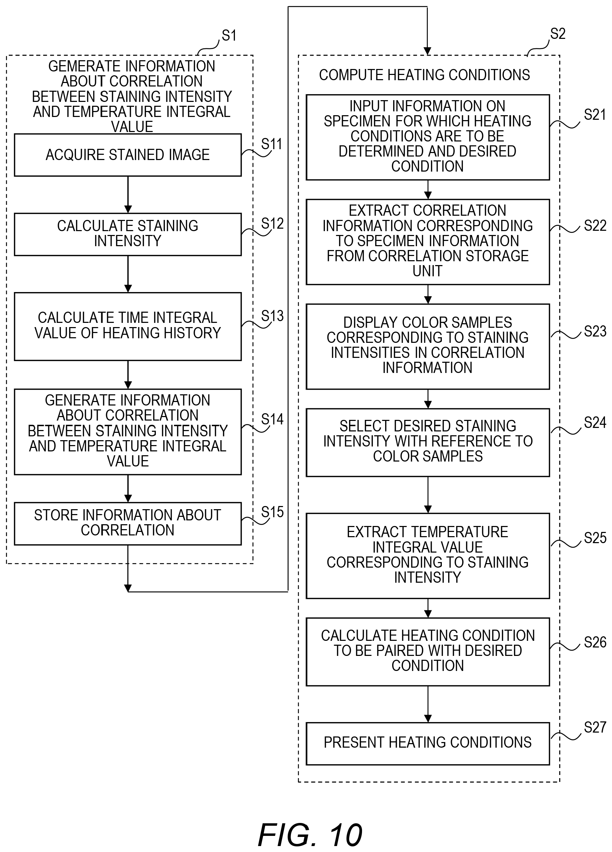

FIG. 10 is a flowchart showing a heating condition presentation method.
[FIG. 11]

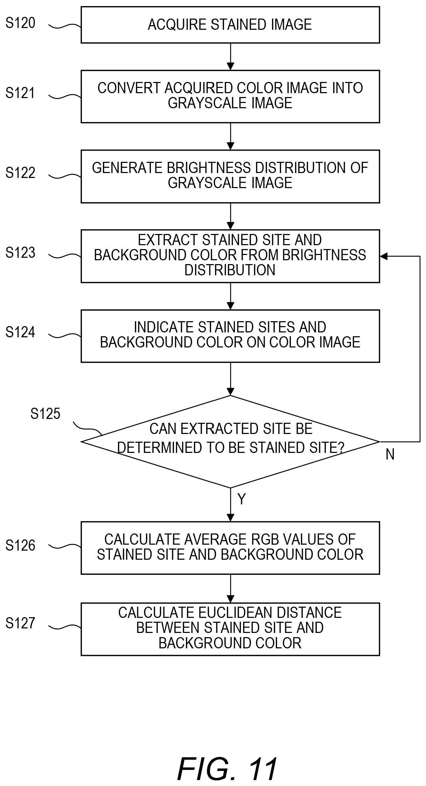

FIG. 11 is a flowchart showing details of image processing of a staining intensity calculation unit.
[FIG. 12]

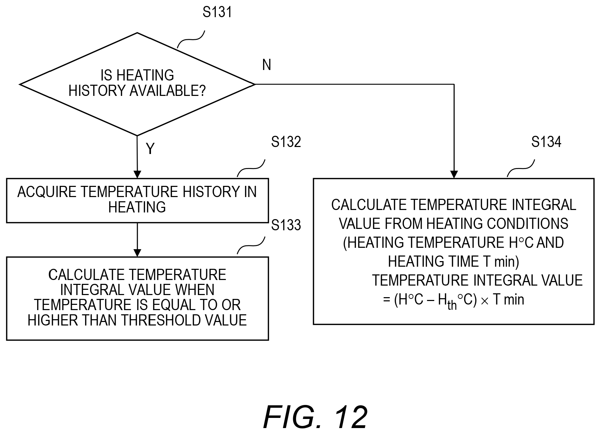

FIG. 12 is a flowchart showing details of processing of a heating history acquisition unit and a temperature integral calculation unit.

[Description of Embodiments]

[First Embodiment]

This disclosure relates to immunohistochemical staining, which involves heating (antigen activation) as a pre-staining treatment for the staining target. This embodiment describes a heating condition presentation device and a heating condition presentation method for presenting optimal heating conditions for the staining target the user (e.g., a clinical laboratory technician). The inventors conducted extensive research into heating conditions for attaining an optimal staining intensity in immunohistochemical staining, and found that a correlation is obtained between the intensity of the stain in a stained specimen and the time integral value of the temperature history (hereinafter referred to as heating history) in the heating as a pre-staining treatment.

FIG. 1 is a diagram showing the correlation between the intensity of the stain in a stained specimen and the time integral value of the heating history (hereinafter referred to as temperature integral value) in the pre-staining treatment. As shown in FIG. 1, there is a positive correlation between the intensity of the stain in a stained specimen and the temperature integral value.

FIG. 2 is a diagram for explaining the definition of the temperature integral value. FIG. 2 provides a graph of a heating history; the horizontal axis represents time (in minutes) and the vertical axis represents temperature (in degrees Celsius). The temperature integral value of the heating history is defined as the area of the region where the temperature is equal to or higher than a threshold value $H_{th}$°C in the heating history curve (the area of the region surrounded by the heating history curve and the straight line passing through the threshold value $H_{th}$°C and parallel to the horizontal axis), which is hatched with slant lines.

Although the temperature for the threshold value $H_{th}$°C is not specifically limited, it can range from 80°C to 95°C or from 90°C to 95°C, for example, from the view point of effective antigen activation.

In this embodiment, the method for presenting heating conditions is roughly separated into two steps. The first step is preparing information about a correlation between staining intensity and temperature integral value, including an approximate formula for the correlation, for each detection target (staining target). The second step is computing heating conditions (a combination of a heating temperature and a heating time) to attain an optimal staining intensity for a given staining target with the prepared information about a correlation and presenting the heating conditions to the user. The heating condition presentation device in this embodiment described in the following is configured to perform these two steps.

<Configuration Example of Heating Condition Presentation Device>

FIG. 3 is a functional block diagram of a heating condition presentation device (information processing device) 1 in the first embodiment. The heating condition presentation device 1 is a device that performs information processing to present optimal heating conditions for a staining target to the user. The heating condition presentation device 1 is implemented by a general-purpose computer such as a workstation, a personal computer, a tablet terminal, or a smartphone. The heating condition presentation device 1 includes memories (e.g., a ROM and a RAM), a processor (e.g., a CPU or a GPU), a storage device for storing programs, and a bus connecting these, although all of these are not shown.

As illustrated in FIG. 3, the heating condition presentation device 1 includes an operating unit 2, a display unit 3, a web interface 4, a correlation database creation unit 10, a correlation storage unit 20, and a heating condition computing unit 30 as software modules (functions implemented by various programs deployed to the memories) to be executed by the processor.

The operating unit 2 is implemented by input devices such as a keyboard, a mouse, a touch panel, and various switches. The operating unit 2 is operated by the user; it receives input of a variety of information and inputs the information to the correlation database creation unit 10 and the heating condition computing unit 30.

The display unit 3 generates a variety of image data based on the display signals input from the correlation database creation unit 10 and the heating condition computing unit 30 and displays the images on a display device. Examples of the display device include a liquid crystal display (LCD), an EL display, and a CRT display.

The web interface 4 has a function to perform communication connection through a web browser. The web interface 4 can be implemented by a modem (and a driver) and a communication module. The heating condition presentation device 1 is configured to be able to connect to a stained image database 41, an imaging device 42, and a staining device 43, which are located externally, through the web interface 4 and the Internet.

The correlation database creation unit 10 generates information about a correlation between staining intensities and temperature integral values of stained specimens, including approximate formulae for the correlations. The details of the method for the correlation database creation unit 10 to generate information about a correlation will be described later. The correlation database creation unit 10 is connected to a stained image database 41 of an external institution, an imaging device 42, and a staining device 43 through the web interface 4 and a communication network such as the Internet.

The stained image database 41 is a free public database provided by, for example, the Japanese Society of Pathology in Japan, the Digital Pathology Association (DPA) in the United States, or UKNEQAS in the United Kingdom. The stained image database 41 stores images of stained specimens collected from medical institutions and educational and research institutions as high-resolution digital images (by whole slide imaging (WSI)).

The imaging device 42 is a device for generating an image of a stained specimen prepared by the staining device 43. Examples of the imaging device 42 include a digital camera, an imaging sensor (e.g., a charge-coupled device (CCD) image sensor or a complementary metal-oxide semiconductor (CMOS) image sensor), and the like.

The staining device 43 is a device for the user to stain the detection target (the staining target) to make a stained specimen. The staining device 43 includes a storage device for storing the heating history in the pre-staining treatment onto the staining target.

The correlation storage unit 20 stores information about a correlation between staining intensity and temperature integral value for each detection target. The correlation storage unit 20 can be implemented by an internal storage or an external storage, for example. Specifically, the correlation storage unit 20 can be implemented by a storage device or an information storage medium such as a flash memory, a hard disk drive (HDD), a solid-state drive (SSD), a storage class memory (SCM), an embedded multi-media card (eMMC), an optical disk, or a magnetic tape, and a reading device therefrom. The storage device for implementing the correlation storage unit 20 can be the same as or different from the storage device for storing programs.

The configuration of the correlation storage unit 20 is not limited to the one installed inside the heating condition presentation device 1. Alternatively, the correlation storage unit 20 can be placed away from the heating condition presentation device 1 or implemented as a database or an on-line storage connected to the heating condition presentation device 1 via a network. Then, the information about correlations between staining intensity and temperature integral value can be shared by a plurality of devices, reducing the time and effort to generate information about correlations in the correlation database creation unit 10.

The heating condition computing unit 30 retrieves information about a correlation between staining intensity and temperature integral value stored in the correlation storage unit 20 and computes heating conditions for the staining target based on the information about the correlation. The details of the method for the heating condition computing unit 30 to compute heating conditions will be described later.

<Processing in Correlation Database Creation Unit 10>

The correlation database creation unit 10 includes a stained image acquisition unit 11, a staining intensity calculation unit 12, a heating history acquisition unit 13, a temperature integral calculation unit 14, and a correlation generation unit 15.

The stained image acquisition unit 11 acquires images of stained specimens (stained images). The stained image acquisition unit 11 can access the external stained image database 41 through the web interface 4 and the Internet. By logging in to the stained image database 41, the user can select a stained image of a desired detection target and download it from the stained image database 41 to the stained image acquisition unit 11. Note that the acquisition source of stained images is not limited to the stained image database 41. Stained images can also be acquired from the imaging device 42 operated by the user.

FIG. 4 is a diagram for explaining image processing of the staining intensity calculation unit 12. The staining intensity calculation unit 12 processes a stained image acquired from the stained image acquisition unit 11 to calculate the staining intensity. As illustrated in FIG. 4, the processing of the staining intensity calculation unit 12 includes the following three steps S100, S200, and S300.

(Step S100)

The staining intensity calculation unit 12 converts a color stained-image into a grayscale one.

(Step S200)

The staining intensity calculation unit 12 generates a brightness distribution of the stained image converted into a grayscale one. Taking an example where a general-purpose staining reagent DAB is used in staining, DAB shows a peak closer to the black in the brightness distribution because DAB is dark brown. Contrarily, the background color shows a peak closer to the white because the background color is nearly white. Hence, the staining intensity calculation unit 12 regards a peak appearing closer to the black as the stained site and a peak appearing closer to the white as the background color to extract sites corresponding to those peaks from the stained image.

(Step S300)

The staining intensity calculation unit 12 selects, for example, 10 or more pixels in each of the extracted stained site and background color from the color stained-image and acquires their RGB values. And then, the staining intensity calculation unit 12 calculates the averages of the color information for each of red, green, and blue.

After the foregoing image processing, the staining intensity calculation unit 12 calculates the staining intensity by the following formula (I), defining the strain intensity as the Euclidean distance in the RGB space (the distance between the stained site (after staining) and the background color (before staining)):

[Math. 1]

Staining intensity

$$= \sqrt{\left(R_{stained\ site} - R_{background\ color}\right)^2 + \left(G_{stained\ site} - G_{background\ color}\right)^2 + \left(B_{stained\ site} - B_{background\ color}\right)^2}$$

(I)

Returning to the description of FIG. 3, the heating history acquisition unit 13 accesses the stained image database 41 or the staining device 43 through the web interface 4 and the Internet to acquire the heating history related to the stained image. In the case of acquiring the heating history of a stained specimen prepared by the staining device 43, the heating history acquisition unit 13 can acquire a heating history like the one shown in FIG. 2.

In the case of acquiring the stained image from the external stained image database 41, it is difficult to acquire a heating history like the one shown in FIG. 2. Accordingly, the heating history acquisition unit 13 acquires information on the heating conditions (the heating temperature H°C and the heating time T min) accompanying the stained image from the stained image database 41.

The temperature integral calculation unit 14 calculates a temperature integral value based on the acquired heating history. In the case of acquiring the heating history from the staining device 43, the temperature integral calculation unit 14 integrates the heating history over time, defining the area of the region hatched by slanted lines where the temperature is equal to or higher than the threshold value $H_{th}$°C as the temperature integral value as illustrated in FIG. 2.

In the case of acquiring the stained image and heating conditions from the external stained image database 41, the available information on the heating conditions is more likely to be only the heating temperature (H°C) and the heating time (T min). Accordingly, in the case of acquiring heating conditions from the stained image database 41, the temperature integral calculation unit 14 calculates the temperature integral value by the following formula (II):

$$\text{Temperature integral value} = (H - H_{th}) \times T \qquad (II)$$

where H represents heating temperature (°C), $H_{th}$ represents the threshold value (°C), and T represents heating time (min).

FIG. 5 is a diagram for explaining the temperature integral value in the case of acquiring heating conditions from the stained image database 41. FIG. 5 shows a hypothetical heating history curve by a broken line. The temperature integral value calculated by the formula (II) is the area of the region hatched by slanted lines in FIG. 5.

FIGS. 6A and 6B are diagrams for explaining the processing of the correlation generation unit 15. The correlation generation unit 15 generates information about a correlation between staining intensity and temperature integral value, including an approximate formula for the correlation, for each detection target and stores the generated information about the correlations to the correlation storage unit 20. In this processing, the correlation generation unit 15 first generates a graph (scatter diagram) having a vertical axis (Y-axis) representing the staining intensity calculated by the staining intensity calculation unit 12 and a horizontal axis (X-axis) representing the temperature integral value calculated by the temperature integral calculation unit 14 and plots the relations between a staining intensity and a temperature integral value for more than ten points, for example. In other words, the above-described processing of each unit of the correlation database creation unit 10 is performed on ten or more stained images.

Next, the correlation generation unit 15 generates an approximated curve of the plot and a correlation approximate formula (III), as illustrated in FIG. 6B.

$$Y = aX + b \qquad (III)$$

where Y represents the staining intensity, X represents the temperature integral value, and a and b represent coefficients.

As illustrated in FIG. 6B, the correlation generation unit 15 may generate not only the correlation approximate formula (III) but also color samples corresponding to staining intensities (information about the correlation) and stores the correlation graph including the color samples to the correlation storage unit 20. Displaying the correlation graph including the color samples (information about the correlation) on the display device enables the user to visually and easily grasp the correlation.

The above-described processing of the correlation database creation unit 10 can be performed at the manufacturer of the heating condition presentation device 1 before shipment or at the user's site after distribution of the heating condition presentation device 1. Even in the case where the correlation database creation unit 10 generates information about correlations before shipment of the heating condition presentation device 1, the correlation database creation unit 10 can further generate information about a correlation after distribution, using the stained images acquired by taking images of stained specimens prepared by the user with the imaging device 4, and update the correlation storage unit 20 with it. The correlation storage unit 20 can be updated as described above not only in the case of the configuration where the correlation storage unit 20 is included within the heating condition presentation device 1 but also in the case of the configuration where the correlation storage unit 20 is accessible from the heating condition presentation device 1 via the Internet.

<Processing in Heating Condition Computing Unit 30>

Returning to the description of FIG. 3, the heating condition computing unit 30 computes heating conditions for a staining target using the information about a correlation between staining intensity and temperature integral value generated by the correlation database creation unit 10. As illustrated in FIG. 3, the heating condition computing unit 30 includes an input unit 31, a correlation extraction unit 32, a staining intensity selection request unit 33, a computing unit 34, and a heating condition presentation unit 35. The input unit 31 receives user input of parameters, including information on the specimen for which heating conditions are to be determined and the user's desired heating temperature or heating time. The correlation extraction unit 32 accesses the correlation storage unit 20 and extracts information about a correlation between staining intensity and temperature integral value corresponding to the input specimen information. The staining intensity selection request unit 33 makes the display unit 3 display color samples included in the extracted information about a correlation and requests the user to select a desired staining intensity. The computing unit 34 calculates a temperature integral value corresponding to the selected staining intensity using the correlation approximate formula and computes heating conditions corresponding to the information input to the input unit 31. The heating condition presentation unit 35 generates an image to be displayed based on the computed heating conditions and displays it with the display unit 3.

FIG. 7 is a diagram for explaining the processing of the input unit 31, showing input screens for the parameters. As illustrated in FIG. 7, the input unit 31 makes the display unit 3 to display parameter input screens to receive a user's input operation through the operating unit 2. As the examples of such parameter input screens, a screen to input information on the specimen for which heating conditions are to be determined, a screen to input a heating temperature desired by the user, and a screen to input a heating time desired by the user are shown.

The specimen information input screen has drop-down lists for selecting the staining reagent to be used for staining and the protein to be detected. The heating temperature input screen and the heating time input screen each have a drop-down list of options. Alternatively, the heating temperature input screen and the heating time input screen can include an input box to input a desired value for the heating temperature or heating time. The input screens can be configured in such a manner that a heating time cannot be input if the user inputs a desired heating temperature and contrarily, a heating temperature cannot be input if the user inputs a desired heating time. Although FIG. 7 provides an example where specimen information and a desired heating temperature or heating time can be input, the input unit 31 can be configured to display only the specimen information input screen.

The correlation extraction unit 32 extracts information about a correlation between staining intensity and temperature integral value corresponding to the specimen information (the staining reagent and the detection target) selected through the input unit 31. The correlation extraction unit 32 retrieves the correlation graph including color samples (FIG. 6B) together with the correlation approximate formula (III).

FIG. 8 is a diagram for explaining the processing of the staining intensity selection request unit 33, showing a screen to select a staining intensity. The staining intensity selection request unit 33 makes the display unit 3 display color samples as shown in FIG. 8 to receive a user's selection operation through the operating unit 2. The color samples are displayed gradationally (divided) in blocks depending on the color shade attained by the staining intensity. The user selects a desired staining intensity with reference to these color samples and clicks a block showing the color shade corresponding to the staining intensity on the screen. The color intensity selection screen may include a text such as "Please select a desired staining intensity."

FIG. 9 is a diagram for explaining the processing of the computing unit 34. As shown in FIG. 9, the computing unit 34 calculates a temperature integral value (X) corresponding to the staining intensity (Y) selected by the user, using the staining intensity - temperature integral value correlation approximate formula (III) extracted at the correlation extraction unit 32. Then, the computing unit 34 substitutes the obtained temperature integral value to the formula (II) to obtain a heating temperature H°C or a heating time T min. For example, in the case where the user inputs a desired heating temperature H°C to the input unit 31, a heating time T min to be paired with it is calculated. In another case where the user inputs a desired heating time T min to the input unit 31, a heating temperature H°C to be paired with it is calculated. In still another case where the user only inputs specimen information through the parameter input screen (FIG. 7), the computing unit 34 may generate a list of combinations of a heating temperature H°C and a heating time T min satisfying the formula (II) for the temperature integral value.

The heating condition presentation unit 35 generates an image to be displayed based on the heating condition (the heating temperature H°C or the heating time T min) calculated by the computing unit 34 and displays it with the display unit 3. The heating conditions for the staining target can be thus presented to the user. The user sets the heating device (not shown) in accordance with the heating conditions presented by the heating condition presentation device 1 to apply a pre-staining treatment.

<Example of Processing in Heating Condition Presentation Device 1>

FIG. 10 is a flowchart illustrating the procedure of a heating condition presentation method to be executed by the heating condition presentation device 1. The steps for presenting heating conditions can be roughly separated into two steps. First, in Step S1, the correlation database creation unit 10 generates information about a correlation between staining intensity and temperature integral value and stores it in the correlation storage unit 20. In Step S2, the heating condition computing unit 30 computes heating conditions based on the information about a correlation retrieved from the correlation storage unit 20, and presents the heating conditions to the user. Hereinafter, Steps S1 and S2 are each described in detail.

(Step S1: Generating information about a correlation)

The processing of generating information about a correlation between staining intensity and temperature integral value is executed in the correlation database creation unit 10 when the manufacturer or the user of the heating condition presentation device 1 inputs an instruction to start generating information about a correlation through the operating unit 2. The following describes the case where the user triggers the execution of the processing to generate information about a correlation.

(Step S11)

The stained image acquisition unit 11 receives an operation by the user to acquire a stained image. For this event, the stained image acquisition unit 11 may generate a text prompting the user to acquire a stained image and send the text to the display unit 3 to show it on the display device. The user accesses the stained image database 41 via the Internet with the stained image acquisition unit 11. The user extracts a stained image of the detection target and downloads it from the stained image database 41 to the stained image acquisition unit 11. Alternatively, the user can acquire a stained image from the imaging device 42 the user is in use or accessible to. The acquisition of a stained image is not limited to the foregoing examples, but a stained image provided in an academic paper or a reagent manufacturer can also be used. In any cases of acquiring a stained image, it is necessary to make sure that heating conditions accompanying the stained image exist.

(Step S12)

The staining intensity calculation unit 12 receives the acquired stained image from the stained image acquisition unit 11. The staining intensity calculation unit 12 processes the stained image to calculate the staining intensity.

FIG. 11 is a flowchart showing details of the image processing of the staining intensity calculation unit 12. The image processing in FIG. 11 (Steps S120 to S127) is performed in accordance with the software stored in the staining intensity calculation unit 12.

At Step S120, the staining intensity calculation unit 12 acquires the stained image acquired by the stained image acquisition unit 11. At Step S121, the staining intensity calculation unit 12 converts the color stained-image into a grayscale one. At Step S122, the staining intensity calculation unit 12 generates a brightness distribution of the converted grayscale image. Since DAB, which is commonly used as a staining reagent, is dark brown, it shows a peak closer to the black in the brightness distribution. In contrast, the background color is nearly white and therefore, it shows a peak closer to the white. Hence, at Step S123, the staining intensity calculation unit 12 extracts the peak appearing closer to the black in the brightness distribution as the stained site and the peak appearing closer to the white as the background color. At Step S124, the staining intensity calculation unit 12 indicates the extracted stained site and background color so that they can be recognized on the color image, and sends the image to the display unit 3 to display it on the display device. At this time, the regions extracted as stained sites can be indicated by circles on the color image, for example, as shown in Step S300 in FIG. 4.

At Step S125, the staining intensity calculation unit 12 sends the display unit 3 data for the above-described color image and the image of a dialog box for the user to input his/her determination about whether the extraction of stained sites has any problem to display them on the display device. Through this configuration, the staining intensity calculation unit 12 receives input about the user's determination about whether the extraction results of stained sites are correct. For example, the dialog box can include a text "Is the extraction of stained sites appropriate?" and a selection box between "Yes" and "No". If the user determines that the extraction of stained sites is correct and inputs "Yes", the processing proceeds to Step S126. If the user determines that the extraction of stained sites is incorrect and inputs "No", the processing returns to Step S123. If the processing returns to Step S123, the staining intensity calculation unit 12 performs extraction of the stained site in the brightness distribution again. At this time, the staining intensity calculation unit 12 may display the brightness distribution of the grayscale image to allow the user to manually select the stained site while viewing the brightness distribution.

At Step S126, the staining intensity calculation unit 12 selects, for example, 10 or more pixels in each of the extracted stained site and background color from the color stained-image, acquires their RGB values, and calculates the averages of the color information for each of red, green, and blue.

At Step S127, the staining intensity calculation unit 12 calculates the staining intensity by the aforementioned formula (I), defining the staining intensity as the Euclidean distance in the RGB space (the distance between the stained site (after staining) and the background color (before staining)).

(Step S13)

Returning to the description of FIG. 10, at Step S13, the heating history acquisition unit 13 accesses the stained image database 41 or the staining device 43 to acquire the heating history related to the stained image. The temperature integral calculation unit 14 calculates the time integral value of the heating history (temperature integral value).

FIG. 12 is a flowchart showing details of the processing of the heating history acquisition unit 13 and the temperature integral calculation unit 14. The processing in FIG. 12 (Steps S131 to S134) is performed in accordance with the software stored in the heating history acquisition unit 13 and the temperature integral calculation unit 14.

At Step S131, the heating history acquisition unit 13 determines whether a heating history like the one shown in FIG. 2 is available. Specifically, in the case where the stained specimen is prepared by the staining device 43 and the stained image is prepared by the imaging device 42, the heating history related to the stained image can be acquired from the staining device 43 and accordingly, the heating history acquisition unit 13 determines that a heating history is available (Yes). In the other case where the stained image is acquired from an external database such as the stained image database 41, the heating history acquisition unit 13 determines that a heating history is unavailable (No). If the heating history is available (Yes), the processing proceeds to Step S132. If the heating history is unavailable (No), the processing proceeds to Step S134.

At Step S132, the heating history acquisition unit 13 accesses the staining device 43 and acquires a heating history like the one illustrated in FIG. 2. At Step S133, the temperature integral calculation unit 14 calculates the area of the region where the temperature is equal to or higher than the threshold value $H_{th}$°C in the heating history curve (the region hatched with slanted lines in FIG. 2) as a temperature integral value.

In the other case where the stained image is acquired from the stained image database 41, the heating history acquisition unit 13 acquires the heating conditions (the heating temperature H°C and the heating time T min) accompanying the stained image as an alternative heating history at Step S134. The temperature integral calculation unit 14 calculates the temperature integral value by the aforementioned formula (II).

(Step S14)

Returning to the description of FIG. 10, the correlation generation unit 15 receives the staining intensity calculated by the staining intensity calculation unit 12 at Step S12 and the temperature integral value calculated by the temperature integral calculation unit 14 at Step S13. The correlation generation unit 15 generates a graph having a vertical axis (Y-axis) representing staining intensity and a horizontal axis (X-axis) representing time integral value and plots the relation between the staining intensity and the temperature integral value (FIG. 6A). The correlation generation unit 15 generates a correlation graph (FIG. 6B) including an approximated curve of the plot and the aforementioned correlation approximate formula (III).

(Step S15)

The correlation generation unit 15 accesses the correlation storage unit 20 and stores the correlation approximate formula (III) and the correlation graph generated at Step S14 as information about a correlation between staining intensity and temperature integral value.

The foregoing has described an embodiment in which the above-described processing of Step S1 to generate information about a correlation is performed at the user's site. Alternatively, the processing of Step S1 may be performed at the manufacturer's site before shipment of the heating condition presentation device 1.

(Step S2: Computing heating conditions)

The processing of computing heating conditions is executed in the heating condition computing unit 30 when the user inputs a request to compute heating conditions through the operating unit 2.

(Step S21)

Upon receipt of a request to compute heating conditions, the input unit 31 sends data for a parameter input screen for the staining target (FIG. 7) to the display unit 3 and displays the screen on the display device to receive input of parameters. The information to be input by the user includes the staining reagent to be used in staining, the protein to be detected, and a desired heating temperature or heating time. In the case where the user inputs a desired heating temperature, a heating time is computed as a heating condition at a subsequent step. In another case where the user inputs a desired heating time, a heating temperature is computed as a heating condition at a subsequent step.

(Step S22)

The correlation extraction unit 32 receives the user's input information from the input unit 31 and accesses the correlation storage unit 20. The correlation extraction unit 32 extracts and downloads the information about a correlation between staining intensity and temperature integral value associated with the input staining reagent and detection target from the correlation storage unit 20.

(Step S23)

The staining intensity selection request unit 33 receives the downloaded information about a correlation from the correlation extraction unit 32. The staining intensity selection request unit 33 generates a staining intensity selection screen (FIG. 8) based on the color samples in the correlation information and sends it to the display unit 3 to display on the display device. The color samples can be configured of a plurality of blocks gradationally showing color shades depending on the staining intensity.

(Step S24)

The staining intensity selection request unit 33 receives the user's selection of a desired staining intensity based on a color sample. At this time, the user clicks the block showing the color shade attained by the desired staining intensity on the image of the color samples.

(Step S25)

The computing unit 34 receives information on the color shade selected by the user from the staining intensity selection request unit 33. The computing unit 34 substitutes the staining intensity corresponding to the selected color shade to the correlation approximate formula (III) to calculate the temperature integral value.

(Step S26)

The computing unit 34 substitutes the calculated temperature integral value to the formula (II) to calculate a heating time to be paired with the desired heating temperature or a heating temperature to be paired with the desired heating time. In the case where the user inputs neither a desired heating time nor a desired heating temperature at Step S21, the computing unit 34 calculates combinations of a heating temperature and a heating time satisfying the formula (II) for the temperature integral value.

(Step S27)

The heating condition presentation unit 35 receives the calculated heating condition (heating temperature or heating time) from the computing unit 34. The heating condition presentation unit 35 generates image data based on the heating conditions and sends it to the display unit 3 to display on the display device. As a result, the calculated heating condition can be presented to the user.

<Summary of First Embodiment>

As described above, the heating condition presentation device (information processing device) 1 in this embodiment includes a memory for storing programs and a processor for retrieving a program from the memory and executing the program. The heating condition presentation device 1 includes a heating condition computing unit 30 as a software module to be executed by the processor. A correlation extraction unit 32 of the heating condition computing unit 30 retrieves information about a correlation between the staining intensity calculated from a stained image of a stained specimen and the time integral value of heating temperature calculated from the heating history of the stained specimen, including an approximate formula for the correlation. The information is held in a correlation storage unit (storage device) 20. A staining intensity selection request unit 33 receives a selection of a desired staining intensity for the staining target. A computing unit 34 calculates a time integral value corresponding to the selected desired staining intensity based on the information about the correlation retrieved from the correlation storage unit 20 and calculates heating conditions for the staining target from the time integral value. A heating condition presentation unit 35 displays the calculated heating conditions with a display unit (display device) 3.

Through this configuration, the user can know the heating conditions to attain an optimal staining intensity for a specimen to be stained without try and error in exploring heating conditions. Therefore, not only reduction in the burden on the user but also appropriate pathological diagnosis based on a good stained image can be achieved.

[Second Embodiment: Example employing Euclidean distance in Lab space as staining intensity]

The first embodiment defines the staining intensity by the Euclidean distance in the RGB space. The staining intensity can be expressed in another color space. The second embodiment defines the staining intensity by the Euclidean distance in the Lab space. The L*a*b* space can be expressed as a three-dimensional space like the RGB space; L represents lightness, a* represents green-red chroma, and b* represents blue-yellow chroma. Compared to the RGB space, the L*a*b* space is close to the human color perception mechanism.

The Euclidean distance in the L*a*b* space can be calculated by the following three steps (i) to (iii), based on the RGB values acquired from a stained image.

(i) Converting RGB values to tristimulus values XYZ

[Math. 2]

$$\begin{pmatrix} X \\ Y \\ Z \end{pmatrix} = \begin{pmatrix} 0.412424 & 0.357579 & 0.180464 \\ 0.212656 & 0.715158 & 0.072186 \\ 0.019332 & 0.119193 & 0.950444 \end{pmatrix} \begin{pmatrix} f(R/255) \\ f(G/255) \\ f(B/255) \end{pmatrix} \qquad (IV)$$

[Math. 3]

$$f(t) = \begin{cases} \left(\dfrac{t+0.055}{1.055}\right)^{2.4} & (t > 0.04045) \\ \dfrac{t}{12.92} & (t \leq 0.04045) \end{cases} \qquad (V)$$

(ii) Converting tristimulus values XYZ to L*a*b* values

[Math. 4]

$$L^* = \begin{cases} 116 \times \left(\frac{Y}{Y_0}\right)^{\frac{1}{3}} - 16 & \left(\frac{Y}{Y_0} > 0.008856\right) \\ 903.29 \times \left(\frac{Y}{Y_0}\right) & \left(\frac{Y}{Y_0} \le 0.008856\right) \end{cases}$$

$$a^* = 500 \times \left(\left(\frac{X}{X_0}\right)^{\frac{1}{3}} - \left(\frac{Y}{Y_0}\right)^{\frac{1}{3}}\right)$$

$$b^* = 200 \times \left(\left(\frac{Y}{Y_0}\right)^{\frac{1}{3}} - \left(\frac{Z}{Z_0}\right)^{\frac{1}{3}}\right) \tag{VI}$$

(iii) Calculating staining intensity (Euclidean distance)

[Math. 5]

$$\Delta E^*_{ab} = \sqrt{(\Delta L^*)^2 + (\Delta a^*)^2 + (\Delta b^*)^2} \tag{VII}$$

At the above-described Step S14, the correlation generation unit 15 can generate a correlation using the staining intensity calculated by the staining intensity calculation unit 12 using the formula (VII). Such staining intensities expressed with L*a*b* values can make the color samples to be presented to the user closer to the colors actually perceived by the user. Accordingly, the user can rightly select a desired staining intensity from the color samples. As a result, the possibility of presenting optimal heating conditions increases, compared to the case using the RGB values.

[Third Embodiment: Example including stained image acquisition unit that takes stained image of specimen]

According to the first embodiment, the stained image acquisition unit 11 accesses the external stained image database 41 or the imaging device 42 via the Internet to acquire a stained image. However, the source to acquire the stained image is not limited to an external device. A configuration such that the stained image acquisition unit 11 has a function to take a stained image or a configuration such that the stained image acquisition unit 11 includes the imaging device 42 can also be employed. Then, a stained image can be acquired within the heating condition presentation device 1, facilitating the processing of the stained image acquisition unit 11. As to the heating history, the same configuration as the one in the first embodiment that acquires a heating history from the external staining device 43 can be employed.

[Modifications]

This disclosure is not limited to the foregoing embodiments but includes various modifications. For example, the foregoing embodiments have been described specifically to clearly explain the disclosure; they are not necessary to include all of the described configurations. Furthermore, a part of one embodiment can be replaced with a configuration of another embodiment. A configuration of an embodiment can be incorporated into a configuration of another embodiment. A part of the configuration of an embodiment can be added to, deleted from, or replaced with a part of the configuration of another embodiment.

[List of Reference Signs]

1     Heating condition presentation device
2     Operating unit
3     Display unit
4     Web interface
10    Correlation database creation unit
11    Stained image acquisition unit
12    Staining intensity calculation unit
13    Heating history acquisition unit

14    Temperature integral calculation unit
15    Correlation generation unit
20    Correlation storage unit
30    Heating condition computing unit
31    Input unit
32    Correlation extraction unit
33    Staining intensity selection request unit
34    Computing unit
35    Heating condition presentation unit
41    Stained image database
42    Imaging device
43    Staining device

**Claims**

1. An information processing device for presenting at least one heating condition for a staining target, the information processing device comprising:

   a memory configured to store a program; and
   a processor configured to retrieve the program from the memory and execute the program,
   wherein the processor is configured to perform the processing of:

   retrieving information about a correlation between a staining intensity of a stained specimen and a time integral value of heating temperature for the stained specimen from a storage device holding the information about the correlation, the information about the correlation including an approximate formula for the correlation between the staining intensity calculated from a stained image of the stained specimen and the time integral value of heating temperature calculated from a heating history of the stained specimen; receiving a selection of a desired staining intensity for the staining target; calculating a time integral value of heating temperature corresponding to the selected desired staining intensity based on the information about the correlation retrieved from the storage device and calculating the at least one heating condition for the staining target from the time integral value; and displaying the calculated at least one heating condition on a display device.

2. The information processing device according to claim 1,

   wherein the storage device further holds color samples corresponding to staining intensities as the information about the correlation, and
   wherein the processor is configured to:

   display the color samples on the display device in the processing of receiving a selection of a desired staining intensity; receive input of information about the staining target and a desired heating temperature or a desired heating time in the processing of calculating at least one heating condition; calculate the time integral value of heating temperature corresponding to the selected desired staining intensity by the approximate formula for the correlation to calculate a heating time to be paired with the input desired heating temperature or a heating temperature to be paired with the input desired heating time as the at least one heating condition in the processing of calculating at least one heating condition; and display the calculated heating time or heating temperature on the display device in the processing of displaying.

3. The information processing device according to claim 1, wherein the processor is configured to:

   obtain the information about the correlation by further executing the processing of:

   acquiring the stained image of the stained specimen; calculating the staining intensity of the acquired stained image; acquiring the heating history of the stained specimen; and calculating the time integral value in the heating history; and

store the information about the correlation to the storage device.

4. The information processing device according to claim 1, wherein the staining intensity is a Euclidean distance between a stained site and a background color in an RGB color space of the stained image.

5. The information processing device according to claim 1, wherein the staining intensity is a Euclidean distance between a stained site and a background color in an $L^*a^*b^*$ color space of the stained image.

6. The information processing device according to claim 4 or 5, wherein the stained site and the background are extracted from a grayscale brightness distribution of the stained image.

7. The information processing device according to claim 3, wherein the processor is configured to access an external stained image database via a communication network to acquire the stained image from the stained image database.

8. The information processing device according to claim 3, wherein the processor is configured to access an external imaging device that has taken the stained image of the stained specimen to acquire the stained image from the imaging device.

9. The information processing device according to claim 3, wherein the processor is configured to access an external stained image database via a communication network to acquire the heating history of the stained specimen from the stained image database.

10. The information processing device according to claim 3, wherein the processor is configured to access an external staining device that has stained the stained specimen to acquire the heating history from the staining device.

11. The information processing device according to claim 1, further comprising:
an imaging device configured to take a stained image of a stained specimen.

12. The information processing device according to claim 1, wherein the at least one heating condition includes a heating temperature and a heating time.

13. The information processing device according to claim 1, wherein the time integral value of heating temperature is calculated by (heating temperature - temperature threshold value) × heating time.

14. The information processing device according to claim 2, wherein the information about the staining target includes names of a staining reagent and a protein.

15. The information processing device according to claim 2, wherein the color samples are displayed gradationally depending on color shades attained by staining intensities.

16. An information processing method to present at least one heating condition for a staining target, the method being implemented by a processor of an information processing device executing a program stored in a memory and comprising:

retrieving, by the processor, information about a correlation between a staining intensity of a stained specimen and a time integral value of heating temperature for the stained specimen from a storage device holding the information about the correlation, the information about the correlation including an approximate formula for the correlation between the staining intensity calculated from a stained image of the stained specimen and the time integral value of heating temperature calculated from a heating history of the stained specimen;
receiving, by the processor, a selection of a desired staining intensity for the staining target;
calculating, by the processor, a time integral value of heating temperature corresponding to the selected desired staining intensity based on the information about the correlation retrieved from the storage device and calculating, by the processor, the at least one heating condition for the staining target from the time integral value; and
displaying, by the processor, the calculated at least one heating condition on a display device.

17. The information processing method according to claim 16,

wherein the storage device further holds color samples corresponding to staining intensities as the information about the correlation, and

wherein the receiving a selection of a desired staining intensity includes displaying the color samples on the display device,

wherein the calculating the at least one heating condition includes:

receiving input of information about the staining target and a desired heating temperature or a desired heating time; and

calculating the time integral value of heating temperature corresponding to the selected desired staining intensity by the correlation approximate formula to calculate a heating time to be paired with the input desired heating temperature or a heating temperature to be paired with the input desired heating time as the at least one heating condition, and

wherein the displaying includes displaying the calculated heating time or heating temperature on the display device.

## FIG. 1

## FIG. 2

FIG. 3

(STEP S100) CONVERT COLOR STAINED IMAGE INTO GRAYSCALE ONE

COLOR          GRAYSCALE

(STEP S200) GENERATE GRAYSCALE BRIGHTNESS DISTRIBUTION AND
EXTRACT STAINED SITE AND BACKGROUND COLOR

(STEP S300) ACQUIRE RGB VALUES OF STAINED SITE AND BACKGROUND COLOR

# FIG. 4

*FIG. 5*

*FIG. 6A*

*FIG. 6B*

| SPECIMEN INFORMATION INPUT SCREEN | HEATING TEMPERATURE INPUT SCREEN | HEATING TIME INPUT SCREEN |
|---|---|---|

STAINING REAGENT ▼

DETECTION TARGET
ALK △
BRAF
CD20
CD3
HER2 ▽

OK     cancel

HEATING TEMPERATURE [°C]
95 △
100
105
110
115 ▽

OK     cancel

HEATING TIME [min]
1 △
3
5
10
15 ▽

OK     cancel

# FIG. 7

STAINING INTENSITY SELECTION SCREEN

STAINING
INTENSITY
WEAK
STRONG

## FIG. 8

Y

Y=aX+b ...CORRELATION APPROXIMATE FORMULA (III)

STAINING INTENSITY

TEMPERATURE INTEGRAL VALUE
$= (H°C - H_{th}°C) \times T$ min ...FORMULA (II)

X

TEMPERATURE INTEGRAL VALUE [°C·min]

## FIG. 9

FIG. 10

S120 — ACQUIRE STAINED IMAGE

S121 — CONVERT ACQUIRED COLOR IMAGE INTO GRAYSCALE IMAGE

S122 — GENERATE BRIGHTNESS DISTRIBUTION OF GRAYSCALE IMAGE

S123 — EXTRACT STAINED SITE AND BACKGROUND COLOR FROM BRIGHTNESS DISTRIBUTION

S124 — INDICATE STAINED SITES AND BACKGROUND COLOR ON COLOR IMAGE

S125 — CAN EXTRACTED SITE BE DETERMINED TO BE STAINED SITE?

N

Y

S126 — CALCULATE AVERAGE RGB VALUES OF STAINED SITE AND BACKGROUND COLOR

S127 — CALCULATE EUCLIDEAN DISTANCE BETWEEN STAINED SITE AND BACKGROUND COLOR

# FIG. 11

S131

IS HEATING
HISTORY AVAILABLE?

N

Y

S132

ACQUIRE TEMPERATURE HISTORY IN
HEATING

S133

CALCULATE TEMPERATURE
INTEGRAL VALUE WHEN
TEMPERATURE IS EQUAL TO OR
HIGHER THAN THRESHOLD VALUE

S134

CALCULATE TEMPERATURE INTEGRAL
VALUE FROM HEATING CONDITIONS
(HEATING TEMPERATURE H°C AND
HEATING TIME T min)
TEMPERATURE INTEGRAL VALUE
$= (H°C - H_{th}°C) \times T$ min

*FIG. 12*

## INTERNATIONAL SEARCH REPORT

| | International application No. |
|---|---|
| | **PCT/JP2024/031780** |

**A.    CLASSIFICATION OF SUBJECT MATTER**

*G01N 33/48*(2006.01)i; *G01N 1/30*(2006.01)i
FI:    G01N33/48 P; G01N1/30

According to International Patent Classification (IPC) or to both national classification and IPC

**B.    FIELDS SEARCHED**

Minimum documentation searched (classification system followed by classification symbols)

G01N33/48; G01N1/30

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | WO 2016/047625 A1 (NICHIREI BIOSCIENCES INC.) 31 March 2016 (2016-03-31) entire text, all drawings, particularly, see claims, paragraphs [0031]-[0076], fig. 4, etc. | 1-17 |
| A | JP 2001-296302 A (UNIV. NIHON) 26 October 2001 (2001-10-26) entire text, all drawings, particularly, see claims, etc. | 1-17 |
| A | JP 2020-527707 A (LUNAPHORE TECHNOLOGIES SA) 10 September 2020 (2020-09-10) see entire text, etc. | 1-17 |
| A | WO 2021/241061 A1 (SONY GROUP CORPORATION) 02 December 2021 (2021-12-02) see entire text, etc. | 1-17 |
| A | JP 2022-037567 A (SCREEN HOLDINGS CO., LTD.) 09 March 2022 (2022-03-09) see entire text, etc. | 1-17 |
| A | JP 2012-032402 A (SHIMADZU CORPORATION) 16 February 2012 (2012-02-16) see entire text, etc. | 1-17 |

☑ Further documents are listed in the continuation of Box C.     ☑ See patent family annex.

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **31 October 2024** | **19 November 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/031780**

| C. | DOCUMENTS CONSIDERED TO BE RELEVANT | |
|---|---|---|
| Category* | Citation of document, with indication, where appropriate, of the relevant passages | Relevant to claim No. |
| A | JP 7283642 B1 (NITTO BOSEKI CO., LTD.) 30 May 2023 (2023-05-30)<br>see entire text, etc. | 1-17 |

Form PCT/ISA/210 (second sheet) (July 2022)

**INTERNATIONAL SEARCH REPORT**
Information on patent family members

International application No.

**PCT/JP2024/031780**

| Patent document cited in search report | | | Publication date (day/month/year) | Patent family member(s) | | | Publication date (day/month/year) |
|---|---|---|---|---|---|---|---|
| WO | 2016/047625 | A1 | 31 March 2016 | US entire text, all drawings, particularly, see claims, paragraphs [0106]-[0151], FIG. 4, etc. | 2017/0292899 | A1 | |
| | | | | US | 2019/0025164 | A1 | |
| | | | | EP | 3199936 | A1 | |
| | | | | JP | 2020-106539 | A | |
| JP | 2001-296302 | A | 26 October 2001 | (Family: none) | | | |
| JP | 2020-527707 | A | 10 September 2020 | US see entire text, etc. | 2020/0353467 | A1 | |
| | | | | US | 2023/0372928 | A1 | |
| | | | | WO | 2019/012005 | A1 | |
| | | | | EP | 3651902 | A1 | |
| | | | | EP | 3427829 | A1 | |
| | | | | JP | 2023-162212 | A | |
| WO | 2021/241061 | A1 | 02 December 2021 | US see entire text, etc. | 2023/0184639 | A1 | |
| JP | 2022-037567 | A | 09 March 2022 | US see entire text, etc. | 2022/0067938 | A1 | |
| | | | | EP | 3961563 | A1 | |
| JP | 2012-032402 | A | 16 February 2012 | US see entire text, etc. | 2010/0075372 | A1 | |
| | | | | WO | 2008/038813 | A1 | |
| | | | | EP | 2071313 | A1 | |
| JP | 7283642 | B1 | 30 May 2023 | US see entire text, etc. | 2024/0085306 | A1 | |
| | | | | WO | 2023/053574 | A1 | |
| | | | | EP | 4410952 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020527707 A

**Non-patent literature cited in the description**

- *Aichi Prefecture Clinical Testing Quality Control Survey*, 2014, 217-235